# EUROPEAN PATENT APPLICATION

(11) **EP 3 026 010 A1**
(43) Date of publication of application: **01.06.2016**
(21) Application number: 14829914.2
(22) Date of filing: 18.07.2014
(51) Int. Cl.: B81C 3/00, B01J 19/00, B29D 24/00, B81B 1/00, G01N 37/00, G03F 7/038

(54) **MICROFLUIDIC DEVICE AND PROCESS FOR PRODUCING SAME, AND PHOTOSENSITIVE RESIN COMPOSITION FOR FORMING FLOW PATH**

(30) Priority: 24.07.2013 JP 2013153385
(71) Applicant: JSR Corporation, Minato-ku Tokyo 105-8640 (JP)
(72) Inventor: HIEDA, Katsuhiko, Tokyo 105-8640 (JP); INOMATA, Katsumi, Tokyo 105-8640 (JP); MIYAZAKI, Tomokazu, Tokyo 105-8640 (JP); DOI, Takashi, Tokyo 105-8640 (JP); KUBO, Hiroto, Tokyo 105-8640 (JP)
(74) Representative: TBK
(86) International application number: PCT/JP2014/069157
(87) International publication number: WO 2015/012212

(57) **Abstract**

An object of the invention is to provide microfluidic devices having a channel that is microscopic but is free from the risk of the attachment of substrates. A microfluidic device includes a channel in a body, and the channel has a patterned resin composition layer formed from a photosensitive resin composition including an oxiranyl group-containing compound, an oxetanyl group-containing compound and a photoacid generator.

## Description

### TECHNICAL FIELD

The present invention relates to a process for producing microfluidic devices, to a microfluidic device, and to a channel-forming photosensitive resin composition.

### BACKGROUND ART

Microfluidic devices (also referred to by other names such as microchannel devices, microchannel chips and microreactors) are compact devices in the form of chips including a microchannel for the passage of a fluid such as liquid or gas, a valve, and an inlet and an outlet for the fluid. After their development, such devices are being applied to a wide range of uses such as separation analysis, sensor and chemical reaction fields of various substances.

The application of microfluidic devices that is of interest in recent years is to the high-throughput screening of products such as medicines and pesticides in which the devices are used for the separation analysis and the synthesis of biological substances such as proteins, nucleic acids including DNA and RNA, and sugar chains.

Microfluidic devices include a capillary-like microchannel in a body made of a material such as glass or resin, and also include a pumping mechanism for delivering a substrate through the inside of the channel, and a detection mechanism.

As known in the art, channels in such microfluidic devices are produced using energy ray-curable resin compositions (photosensitive resin compositions) (Patent Literature 1).

### CITATION LIST

### PATENT LITERATURE

Patent Literature 1: JP-A-2009-501908

### SUMMARY OF INVENTION

### TECHNICAL PROBLEM

As is the case in semiconductor integrated circuits, the reduction in the size of channels in microfluidic devices allows reactions to be performed quickly and efficiently. For example, the diffusion of a substrate flowing in a channel ultimately depends on the diffusion area and hence the time required for the diffusion is proportional to the square of the diffusion distance. With decreasing diameter of the channel, the diffusion distance is decreased and the substrate can be diffused more quickly. When the reaction is carried out while performing heating, heat can be transferred more quickly with decreasing diameter of the channel because the thermal conductivity is irreversely proportional to the sectional area through which the heat is transferred.

As described above, small channels allow the reaction of substrates to be performed quickly and thus enhance the performance of the microfluidic devices. When a channel is produced using a photosensitive resin composition as in Patent Literature 1, the crosslinking reaction of a patterned film formed by photoexposure and development is interrupted for a time in stage B (in a semi-cured state) in order to arrange a cover layer thereon. Thus, it is often the case that after the arrangement of the cover layer, the curing of the patterned film fails to complete or to bond the cover layer.

It is therefore an object of the present invention to provide a process for producing microfluidic devices which have an improved channel formed from a photosensitive resin composition, and to provide such a microfluidic device and a channel-forming photosensitive resin composition used for the formation of such channels.

### SOLUTION TO PROBLEM

Some of the configurations of the present invention are as described below.
[1] A microfluidic device including a channel in a body,
   the channel having a patterned resin composition layer formed from a photosensitive resin composition including an oxiranyl group-containing compound, an oxetanyl group-containing compound and a photoacid generator.
[2] A microfluidic device including a channel in a body,
   the channel being defined by a support; a patterned resin composition layer formed from a photosensitive resin composition including an oxiranyl group-containing compound, an oxetanyl group-containing compound and a photoacid generator; and a lid covering a groove defined by the support and the patterned resin composition layer.
[3] The microfluidic device described in [1] or [2], wherein the photosensitive resin composition further includes an acid diffusion-controlling agent.
[4] The microfluidic device described in [2], wherein at least one selected from the support and the lid has a detection element.
[5] A process for producing a microfluidic device including a channel in a body, the process including:
   a step of forming a resin composition layer on a support, the resin composition layer including a photosensitive resin composition including an oxiranyl group-containing compound, an oxetanyl group-containing compound and a photoacid generator,
   a step of selectively irradiating and developing the resin composition layer, and
   a step of arranging a lid on the developed resin composition layer.
[6] A channel-forming photosensitive resin composition used for the formation of a channel in a body of a microfluidic device,
   the channel-forming photosensitive resin composition including an oxiranyl group-containing compound, an oxetanyl group-containing compound and a photoacid generator.

### ADVANTAGEOUS EFFECTS OF INVENTION

The invention can provide microfluidic devices having a channel that is microscopic but is free from the risk of the attachment of substrates, the process thereof, and the channel-forming photosensitive resin composition used for the formation of channels.

### BRIEF DESCRIPTION OF DRAWINGS

[Fig. 1] Fig. 1 is a set of schematic views illustrating a process for producing microfluidic devices according to an embodiment of the invention.
[Fig. 2] Fig. 2 is a set of schematic views illustrating a microfluidic device produced in Examples.

### DESCRIPTION OF EMBODIMENTS

Hereinbelow, embodiments of the present invention will be described sequentially with respect to the inventive channel-forming photosensitive resin compositions, the inventive processes for producing microfluidic devices using the channel-forming photosensitive resin compositions, and the inventive microfluidic devices produced with use of the channel-forming photosensitive resin compositions.

### 1. Channel-forming photosensitive resin compositions

The channel-forming photosensitive resin composition (hereinafter, also written as "resist composition") is a photosensitive resin composition used for the formation of a channel in a body of a microfluidic device. The composition includes an oxiranyl group-containing compound (hereinafter, also written as "oxiranyl compound"), an oxetanyl group-containing compound (hereinafter, also written as "oxetanyl compound") and a photoacid generator.

The channel in the microfluidic device of the invention is formed by applying the resist composition to form a resin composition layer, irradiating and developing the layer to form a patterned resin composition layer in a semi-cured state, and joining the support, the patterned resin composition layer and a lid by curing the semi-cured pattern of the resin composition layer. It is necessary that the semi-curing be effected to such an extent that the patterned resin composition layer does not collapse. Oxiranyl compounds generally have a high curing rate and exhibit excellent curability. By virtue of the addition of an oxiranyl compound to the resist composition, the patterned resin composition layer achieves such a curing degree that the collapse of the layer is avoided.

If the resist composition contains only an oxiranyl compound as the crosslinking agent, the patterned resin composition layer that is obtained does not exhibit fluidity when the patterned resin composition layer is joined together with other members due to the oxiranyl compound having such a high curing rate. Herein, the walls of the patterned resin composition layer formed by irradiation and development directly define the profile of the walls of the channel. In general, increasing the resolution of patterns obtained from resist compositions results in the roughening of the walls of the patterns. Thus, resist compositions containing an oxiranyl compound alone cannot form microscopic channels having smooth walls.

Meanwhile, oxetanyl compounds generally have a low curing rate, and therefore a patterned resin composition layer that is obtained from a resist composition containing only an oxetanyl compound as the crosslinking agent may be collapsed when the patterned resin composition layer is in a semi-cured state. On the other hand, the patterned resin composition layer exhibits fluidity when it is joined together with other members. Specifically, the fluidity allows the walls of the patterned resin composition layer formed by irradiation and development to be smoothed so as to attain the desired profile of the walls of the channel.

In the invention, the combined use of an oxiranyl compound and an oxetanyl compound having different curing rates is probably the reason why the patterned resin composition layer is prevented from collapse when it is in a semi-cured state but still can form smooth walls by exhibiting fluidity when the pattern is joined together with other members. For this reason, the resist composition containing both an oxiranyl compound and an oxetanyl compound is particularly suited for the formation of channels in microfluidic devices.

Hereinbelow, there will be described the components present in the resist compositions, specifically, oxiranyl compounds, oxetanyl compounds and photoacid generators, as well as optional components such as alkali-soluble resins, acid diffusion-controlling agents and other additives which may be added to the resist compositions.

### 1-1. Oxiranyl compounds

The oxiranyl compounds are not limited as long as the compounds have one or more oxiranyl groups and are caused to undergo crosslinking reaction by the action of an acid generated by the irradiation of a photoacid generator. The oxiranyl compounds may be low-molecular compounds or resins. The resinous oxiranyl compounds may have acidic groups such as phenolic hydroxyl groups or carboxyl groups in addition to the oxiranyl groups. Such acidic group-containing resinous oxiranyl compounds may be used as alkali-soluble resins described later. In particular, low-molecular oxiranyl compounds are preferable because the obtainable channels have smooth walls.

Examples of the low-molecular oxiranyl compounds include resorcinol diglycidyl ether, pentaerythritol glycidyl ether, phenyl glycidyl ether, neopentyl glycol diglycidyl ether, 1,6-hexanediol diglycidyl ether, sorbitol polyglycidyl ether, propylene glycol diglycidyl ether and trimethylolpropane triglycidyl ether. Examples of the commercially available low-molecular oxiranyl compounds include DENACOL EX-201, EX-313, EX-314, EX-321, EX-411, EX-511, EX-512, EX-612, EX-614, EX-614B, EX-731, EX-810, EX-911 and EM-150 (all manufactured by Nagase ChemteX Corporation).

Examples of the resinous oxiranyl compounds include phenol novolac epoxy resins, cresol novolac epoxy resins, bisphenol epoxy resins, trisphenol epoxy resins, tetraphenol epoxy resins, phenol-xylylene epoxy resins, naphthol-xylylene epoxy resins, phenol-naphthol epoxy resins, phenol-dicyclopentadiene epoxy resins, alicyclic epoxy resins and aliphatic epoxy resins. Examples of the commercially available resinous oxiranyl compounds include JER 827, JER 828, JER 1001, JER 1003, JER 1055, JER 1007, JER 806, JER 807, JER 4004, JER 4005, JER 152 and JER 157S70 (all manufactured by Japan Epoxy Resins Co., Ltd.), EPICLON 860, EPICLON 1050, EPICLON 830, EPICLON 835, EPICLON N-740, EPICLON N-770, EPICLON N-690 and EPICLON N-695 (all manufactured by DIC Corporation), LCE-21, RE-602S and EOCN-1020 (all manufactured by Nippon Kayaku Co., Ltd.), ADEKA RESIN EP-4080S (manufactured by ADEKA CORPORATION), and CELLOXIDE 2021P, CELLOXIDE 2081, CELLOXIDE 2083, CELLOXIDE 2085, EHPE 3150 and EPOLEAD PB 3600 (all manufactured by Daicel Corporation).

In the resinous oxiranyl compounds, the weight average molecular weight measured by gel permeation column chromatography relative to polystyrenes is usually 1,000 to 100,000.

The acidic group-containing resinous oxiranyl compounds may be resins obtained by polymerizing an acidic group-containing monomer such as methacrylic acid, acrylic acid, hydroxystyrene or isopropenylphenol, an oxiranyl group-containing monomer such as glycidyl acrylate, glycidyl methacrylate, glycidyl acrylate, glycidyl methacrylate, p-vinylphenyl glycidyl ether, 3,4-epoxycyclohexylmethyl acrylate or 3,4-epoxycyclohexylmethyl methacrylate, and optionally an additional monomer such as styrene, p-methoxystyrene or butyl methacrylate.

The oxiranyl compounds may be used singly, or two or more may be used in combination.

The content of the oxiranyl compound in the solid of the resist composition is usually 1 to 80 mass%, and preferably 5 to 40 mass%. With respect to 100 parts by mass of the oxetanyl compound, the content of the oxiranyl compound is usually 0.1 to 100 parts by mass, preferably 1 to 50 parts by mass, and more preferably 5 to 30 parts by mass. The use of the oxiranyl compound in the above amount ensures that microscopic channels having smooth walls may be formed.

### 1-2. Oxetanyl compounds

The oxetanyl compounds are not limited as long as the compounds have one or more oxetanyl groups and are caused to undergo crosslinking reaction by the action of an acid generated by the irradiation of a photoacid generator. The oxetanyl compounds may be low-molecular compounds or resins. The resinous oxetanyl compounds may have acidic groups such as phenolic hydroxyl groups or carboxyl groups in addition to the oxetanyl groups. Such acidic group-containing resinous oxetanyl compounds may be used as alkali-soluble resins described later. In particular, oxetanyl compounds used as alkali-soluble resins are preferable because the obtainable channels have smooth walls.

Examples of the low-molecular oxetanyl compounds include 1,4-bis{[(3-ethyl-3-oxetanyl)methoxy]methyl}benzene, di[2-(3-oxetanyl)butyl] ether, 1,4-bis[(3-ethyloxetan-3-yl)methoxy]benzene, 1,3-bis[(3-ethyloxetan-3-yl)methoxy]benzene, 1,2-bis[(3-ethyloxetan-3-yl)methoxy]benzene, 4,4'-bis[(3-ethyloxetan-3-yl)methoxy]biphenyl, 2,2'-bis[(3-ethyl-3-oxetanyl)methoxy]biphenyl, 3,3',5,5'-tetramethyl[4,4'-bis(3-ethyloxetan-3-yl)methoxy] biphenyl, 2,7-bis[(3-ethyloxetan-3-yl)methoxy]naphthalene, 1,6-bis[(3-ethyloxetan-3-yl)methoxy]-2,2,3,3,4,4,5,5-octaf luorohexane, 1,2-bis[2-{(1-ethyl-3-oxetanyl)methoxy}ethylthio]ethane, 2-butyl-2-ethyl-1,3-0-bis[(3-ethyloxetan-3-yl)methyl]-prop ane-1,3-diol, and 1,4-0-bis[(3-ethyloxetan-3-yl)methyl]-butane-1,4-diol.

Examples of the resinous oxetanyl compounds include phenol novolac OXC (3-ethyl-3-chloromethyloxetane) ether (product name "PNOX" manufactured by TOAGOSEI CO., LTD.) and cresol novolac OXC ether (product name "CNOX" manufactured by TOAGOSEI CO., LTD.).

In the resinous oxetanyl compounds, the weight average molecular weight measured by gel permeation column chromatography relative to polystyrenes is usually 1,000 to 100,000.

The acidic group-containing resinous oxetanyl compounds may be resins obtained by polymerizing an acidic group-containing monomer such as methacrylic acid, acrylic acid, hydroxystyrene or isopropenylphenol, an oxetanyl group-containing monomer such as 3-ethyl-3-acryloxymethyloxetane, 3-(acryloyloxymethyl)-3-phenyloxetane, 3-(2-acryloyloxyethyl)-3-ethyloxetane, 3-(methacryloyloxymethyl)oxetane, 3-(methacryloyloxymethyl)-3-methyloxetane or 3-(methacryloyloxymethyl)-3-ethyloxetane, and optionally an additional monomer such as styrene, p-methoxystyrene or butyl methacrylate.

The oxiranyl compounds may be used singly, or two or more may be used in combination.

The content of the oxetanyl compound in the solid of the resist composition is usually 5 to 95 mass%, and preferably 70 to 90 mass%. The use of the oxiranyl compound in the above amount ensures that microscopic channels having smooth walls may be formed.

### 1-3. Photoacid generators

The photoacid generators are compounds that generate an acid by the action of light. When light is applied to a resin film formed of the inventive photosensitive composition, the photoacid generator in the irradiated regions generates an acid, which induces the crosslinking reaction of the oxiranyl compound and the oxetanyl compound. In this manner, the photoacid generator provides a change in the solubility of the irradiated regions with respect to a developing solution. The change in the solubility of the irradiated regions with respect to a developing solution allows the resin film to be patterned.

Examples of the photoacid generators include those photoacid generators described in JP-A-2010-197996 and JP-A-2010-134387 such as onium salt compounds, halogen-containing compounds, sulfone compounds, sulfonate compounds, sulfonimide compounds and diazomethane compounds.

Specific examples of the photoacid generators include:
onium salt compounds such as diphenyliodonium trifluoromethanesulfonate, diphenyliodonium p-toluenesulfonate, diphenyliodonium hexafluoroantimonate, diphenyliodonium hexafluorophosphate, diphenyliodonium tetrafluoroborate, triphenylsulfonium trifluoromethanesulfonate, triphenylsulfonium p-toluenesulfonate, triphenylsulfonium hexafluoroantimonate, 4-t-butylphenyl diphenylsulfonium trifluoromethanesulfonate, 4-t-butylphenyl diphenylsulfonium p-toluenesulfonate, 4,7-di-n-butoxynaphthyl tetrahydrothiophenium trifluoromethanesulfonate, 4-(phenylthio)phenyldiphenylsulfonium tris(pentafluoroethyl)trifluorophosphate and triphenylsulfonium hexafluoroantimonate;
halogen-containing compounds such as 1,10-dibromo-n-decane, 1,1-bis(4-chlorophenyl)-2,2,2-trichloroethane, phenyl-bis(trichloromethyl)-s-triazine, 4-methoxyphenyl-bis(trichloromethyl)-s-triazine, styryl-bis(trichloromethyl)-s-triazine and naphthyl-bis(trichloromethyl)-s-triazine;
sulfone compounds such as 4-trisphenacylsulfone, mesitylphenacylsulfone and bis(phenacylsulfonyl)methane; sulfonate compounds such as benzoin tosylate, pyrogallol tristrifluoromethanesulfonate, o-nitrobenzyl trifluoromethanesulfonate and o-nitrobenzyl p-toluenesulfonate;
sulfonimide compounds such as N-(trifluoromethylsulfonyloxy)succinimide, N-(trifluoromethylsulfonyloxy)phthalimide, N-(trifluoromethylsulfonyloxy)diphenylmaleimide, N-(trifluoromethylsulfonyloxy)bicyclo[2.2.1]hept-5-ene-2,3 -dicarboximide and N-(trifluoromethylsulfonyloxy)naphthylimide; and
diazomethane compounds such as bis(trifluoromethylsulfonyl)diazomethane, bis(cyclohexylsulfonyl)diazomethane and bis(phenylsulfonyl)diazomethane.

The photoacid generators may be used singly, or two or more may be used in combination. To ensure properties of the photosensitive resin composition such as sensitivity, resolution and pattern shape, the content of the photoacid generator in the resist composition is usually 0.1 to 20 parts by mass with respect to 100 parts by mass of the total of the oxiranyl compound and the oxetanyl compound.

### 1-4. Alkali-soluble resins

The alkali-soluble resins are used when alkaline developing solutions are used for development. The alkali-soluble resins are not limited as long as the resins have acidic groups such as phenolic hydroxyl groups and carboxyl groups. The acidic group-containing oxiranyl compounds and the acidic group-containing oxetanyl compounds may be used as the alkali-soluble resins.

Examples of the alkali-soluble resins include novolac resins, polyhydroxystyrene and copolymers thereof, phenol-xylylene glycol condensation resins, cresol-xylylene glycol condensation resins, phenol-dicyclopentadiene condensation resins and polybenzoxazole precursors. These resins may be used singly, or two or more may be used in combination.

In the alkali-soluble resins, the weight average molecular weight measured by gel permeation column chromatography relative to polystyrenes is usually 1,000 to 1,000,000.

In the case where the acidic group-containing oxiranyl or oxetanyl compounds are used as the alkali-soluble resins, the content thereof in the resist composition is as described hereinabove.

When other types of alkali-soluble resins are used, the content thereof in the solid of the resist composition may be 60 mass% or less, although not limited thereto.

### 1-5. Acid diffusion-controlling agents

The acid diffusion-controlling agents are used to inhibit the acid generated by the irradiation of the photoacid generator from being diffused in the resin composition layer. The inhibition of acid diffusion enhances the ability of the resist composition to be resolved. The enhancement in the resolution performance of the resist composition makes it possible for the resin composition layer to be patterned without any roughening of the walls even at a high resolution. Thus, the formation of channels having smooth walls becomes feasible.

Examples of the acid diffusion-controlling agents include those compounds usually used in resist compositions such as those described in literature such as WO 2009/051088. Specific examples of the acid diffusion-controlling agents include tri(cyclo)alkylamines such as triethylamine, tri-n-propylamine and tri-n-butylamine; aromatic amines such as aniline, diphenylamine, triphenylamine and naphthylamine; quaternary ammonium hydroxides such as tetramethylammonium hydroxide and tetra-n-butylammonium hydroxide; nitrogen-containing heterocyclic compounds such as pyrazine, pyrazole and acridine; and protected amines such as N-t-butoxycarbonyl-di-n-octylamine, N-t-butoxycarbonyl-4,4'-diaminodiphenylmethane, N,N'-di-t-butoxycarbonyl-4,4'-diaminodiphenylmethane, N-t-butoxycarbonylbenzimidazole, N-t-butoxycarbonyl-2-methylbenzimidazole, N-t-butoxycarbonyl-2-phenylbenzimidazole, N-t-butoxycarbonyl-pyrrolidine, N-t-butoxycarbonyl-piperidine and N-t-butoxycarbonyl-4-hydroxy-piperidine. Of these, protected amines are preferable because the use thereof makes it possible to form microscopic channels having smooth walls.

The content of the acid diffusion-controlling agent in the resist composition is 0.1 to 10 parts by mass, and preferably 1 to 5 parts by mass with respect to 100 parts by mass of the photoacid generator.

### 1-6. Other additives

In addition to the aforementioned components, the resist composition of the invention may contain various components such as solvents, adhesion improvers, leveling agents, surfactants, sensitizing agents and inorganic particles while still ensuring that the object and the characteristics of the invention are not impaired.

The solvents are used to enhance the handling properties of the resist composition. Any solvents may be used as long as the components other than the solvents are dissolved or dispersed uniformly. Examples of the solvents include propylene glycol monomethyl ether acetate, propylene glycol monomethyl ether, ethyl lactate, cyclohexanone and γ-butyrolactone.

The resist composition may be prepared by homogeneously mixing the components. To remove undesired substances from the composition, the homogeneous mixture of the components may be filtered through a medium such as a filter.

### 2. Processes for producing microfluidic devices

The process for producing microfluidic devices according to the invention is characterized in that a resin composition layer formed of the photosensitive resin composition described above is patterned to form a channel.

For example, the process includes, in the order named:
a step of forming a resin composition layer on a support (hereinafter, also written as "step (1)"), the resin composition layer including the photosensitive resin composition including an oxiranyl group-containing compound, an oxetanyl group-containing compound and a photoacid generator,
a step of selectively irradiating and developing the resin composition layer (hereinafter, also written as "step (2)"), and
a step of arranging a lid on the developed resin composition layer (hereinafter, also written as "step (3)").

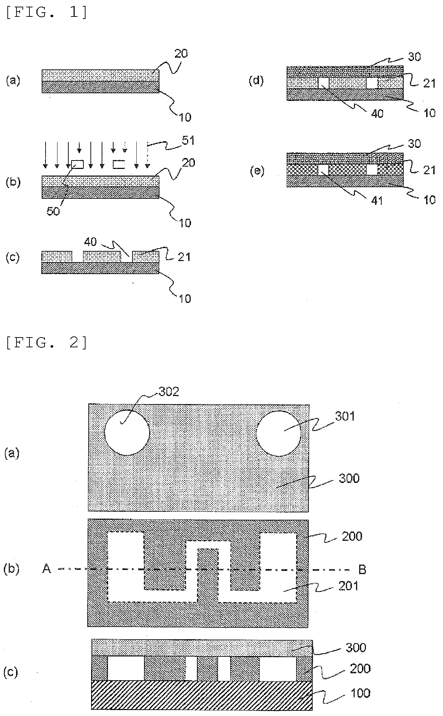

The process for producing microfluidic devices is schematically illustrated in Fig. 1.

### 2-1. Step (1)

In the step (1), a resin composition layer 20 including the aforementioned resist composition is formed on a support 10.

The support 10 may be any material as long as the material allows the resin composition layer to be formed in a uniform thickness without causing any deformation and the material does not react with a substrate flowing in the channel. Examples of the supports 10 include inorganic plates such as silicon, quartz and silicon carbide, and organic polymer plates such as acrylic resin, polystyrene, polycarbonate and polycycloolefin. To allow the inside of the channel to be observed with light, use may be made of a support having excellent transparency to the light. To observe the reaction of a substrate flowing in the channel, a support having a detection element such as a CMOS sensor may be used. The use of the support 10 having a detection element makes it possible to produce smaller microfluidic devices than separately subsequent modulation of the detection element onto the support. The thickness of the support is usually 10 µm to 10 mm. An inlet and/or an outlet may be provided in the support.

For example, the resin composition layer 20 may be provided by directly forming the resin composition layer 20 onto the support 10, or by applying the resist composition onto a release-treated organic film such as PET to form a dry film with a constant thickness and thereafter transferring the dry film onto the support by lamination. In view of the fact that a uniform thickness of the resin composition layer 20 is advantageous for the formation of smaller channels, it is preferable to adopt a method realizing a highly uniform thickness of the film, moreover, to form the resin composition layer 20 directly onto the support 10.

To form the resin composition layer 20 directly onto the support 10, for example, the resist composition may be applied onto the support 10 by an application method such as a spin coating method, an inkjet method, a spray coating method or a bar coating method. Of these methods, a spin coating method is preferable because highly uniform film thickness may be obtained. Prior to the application of the composition to the support 10, the surface on which the composition is applied of the support 10 may be pretreated with a surface treatment agent such as hexamethylsilazane to improve the application properties of the resist composition with respect to the support 10 and to improve the adhesion between the support and the resin composition layer.

After the resist composition has been applied onto the support 10, the film may be heated with, for example, a hot plate or the like to render the film uniform. The heating conditions are usually 60 to 180°C and 10 to 1000 seconds. The thickness of the resin composition layer 20 formed in this manner is usually 0.01 to 1000 µm, and preferably 100 to 500 µm.

### 2-2. Step (2)

In the step (2), the resin composition layer 20 is selectively irradiated and developed.

For example, the layer may be selectively irradiated with use of a contact aligner, a stepper or a scanner through a mask pattern 50 that protects the channel region from light (Fig. 1(b)). Examples of the exposure light 51 include ultraviolet rays and visible rays. Usually, light having a wavelength of 200 to 500 nm is used (for example, i-line wavelength (365 nm)). The irradiance of light of the activation radiation is variable depending on factors such as the types and the proportions of the components in the photosensitive composition, and the thickness of the film. In the case where the i-line wavelength is used as the exposure light, the dose is usually 100 to 1500 mJ/cm².

When the irradiated region has been cured to such an extent that it has sufficient developer resistance while still exhibiting adhesion, the irradiated resin composition layer is developed.

To control the degree of curing of the irradiated region, the exposure may be followed by a baking treatment (hereinafter, also written as "PEB treatment"). The PEB conditions are usually 60 to 180°C.

Thereafter, the irradiated resin composition layer is developed with a developing solution, preferably an alkaline developing solution so as to dissolve away the unexposed region. Consequently, a patterned resin composition layer 21 in a semi-cured state is formed on the support (Fig. 1(c)). The term semi-cured state means that the composition has been cured to such an extent that adhesion is not lost and the gel fraction is not less than 30%. The gel fraction is a ratio in percentage of the mass of a gel remaining after the uncured portion of the resin composition layer is extracted with an appropriate solvent, to the mass of the semi-cured pattern of the resin composition layer.

Examples of the development methods include shower development methods, spray development methods, immersion development methods and puddle development methods. The developing conditions are usually 20 to 40°C and 1 to 10 minutes.

Examples of the alkaline developing solutions include 1 to 10 mass% alkaline aqueous solutions of alkaline compounds such as sodium hydroxide, potassium hydroxide, aqueous ammonia, tetramethylammonium hydroxide and choline. Appropriate amounts of additives such as water-soluble organic solvents such as methanol and ethanol, and surfactants may be added to the alkaline aqueous solutions. After being developed with the alkaline developing solution, the film may be washed with water and dried.

The portions of the patterned resin composition layer 21 that will form the walls of the channel may be surface treated by a method such as electrodeposition or electroless plating to prevent the attachment of a substrate flowing in the channel.

The pattern shapes are not particularly limited. The shapes may be selected appropriately in accordance with the purpose for which the microfluidic device is used, by virtue of the ability of resist compositions to form resin films with various shapes. For example, a pillar pattern may be formed in the case of a pillar-type microfluidic device.

As described in, for example, JP-A-2009-501908, the patterned layer may define an inlet chamber at an end, a channel connected to the inlet chamber, and an absorbent chamber connected to the channel. Other chambers such as a reaction chamber, a detection chamber and a mixing chamber may be provided. The channels do not need to be linear, and the channels and various chambers may be arranged in various configurations such as in a non-linear configuration. The channel system may be composed of a single channel or a plurality of channels. Further, as described in JP-A-2005-329479 and JP-A-2004-130219, a plurality of resin composition layers may be patterned in combination so as to form separate areas such as a detection area, a reaction/separation area and an adjustment area. Furthermore, as described in JP-A-2010-237053, the channel may be curved with a specific curvature so as to prevent the flow from being turbulent.

The width of the pattern is not particularly limited and may be selected appropriately in accordance with the purpose. The width is usually 1 to 1000 µm.

### 2-3. Step (3)

In the step (3), a lid 30 is arranged on the developed resin composition layer.

The lid 30 may be any material that is not deformed and does not react with a substrate flowing in the channel. Examples include those plates described as the supports 10. To allow the inside of the channel to be observed with light, use may be made of a lid 30 having excellent transparency to the light. To observe the reaction of a substrate flowing in the channel, a lid 30 having a detection element such as a CMOS sensor may be used. The incorporation of a detection element into the lid 30 makes it possible to produce smaller microfluidic devices than when the detection element is separately modularized. The thickness of the lid 30 may be similar to the thickness of the support, and is usually 10 µm to 10 mm. To observe the reaction of a substrate flowing in the channel, a support having a detection element such as a CMOS sensor may be used.

The lid 30 may have an inlet through which a substrate is introduced into the channel, and/or an outlet through which the substrate is discharged out of the channel. (These apertures are not shown in the drawing.)

The stack may be exposed to light or heat treated in order to join the support 10, the patterned resin composition layer 21 and the lid 30 into one unit and thereby to form a channel 41 defined by the support 10, the patterned resin composition layer 21 and the lid 30.

The stack is joined into a unit as a result of the semi-cured pattern 21 of the resin composition layer being cured.

For this curing, the treatment such as exposure or heating is performed to induce the crosslinking reaction of the oxiranyl compound and the oxetanyl compound present in the patterned resin composition layer 21. In particular, heat treatment is preferable because the walls of the patterned resin composition layer 21 are fluidized more efficiently.

The heating conditions are not particularly limited, but are usually 100 to 250°C, preferably 150 to 230°C, and 30 minutes to 10 hours. Heating may be performed in two stages to prevent deformation. For example, heating may be performed at a temperature of 50 to 250°C for 30 seconds to 2 hours in the first stage, and may be performed for 20 minutes to 8 hours in the second stage at a temperature equal to or higher than the temperature in the first stage. It is desirable to increase the pressure during the heating. Increasing the pressure makes it possible to bond the lid 30 and the resin composition 21 together more reliably.

After the treatment, features having functions such as liquid delivery and recovery may be added as required.

By virtue of the use of the inventive photosensitive resin composition, the process of the invention can produce microfluidic devices having smooth walls.

As required, features having functions such as liquid delivery and recovery may be added.

In the manner described above, microfluidic devices including a channel in the body may be produced.

### 3. Microfluidic devices

The microfluidic device in an aspect of the invention is characterized in that it includes a channel having a patterned resin composition layer which is formed from a photosensitive resin composition including an oxiranyl group-containing compound, an oxetanyl group-containing compound and a photoacid generator. The microfluidic device in another aspect of the invention includes a channel defined by a support, a patterned resin composition layer and a lid covering a groove defined by the patterned resin composition layer and the support.

For example, such microfluidic devices of the invention may be produced by the process described hereinabove.

In the course of the channel through which a fluid is passed, features are provided such as a mechanism that heats or cools the fluid, a mechanism that dilutes or concentrates the fluid, a mechanism that performs chemical reactions, a mechanism that controls the states of the flow such as the speed of the flow and the branching of the flow, a mechanism that controls operations such as mixing, dissolution and separation, and a detection element that performs electrical or optical measurements.

Details of the support, the resin composition layer, the lid and the channel are as described hereinabove.

In an example of the structures of the microfluidic devices, the thicknesses of the support and the lid are usually about 10 µm to 10 mm, and the thickness of the resin composition layer is usually 0.01 to 1000 µm, and preferably 100 to 500 µm.

The resin composition layer may be a single layer or a stack of layers. The channel is a space surrounded by the support, the patterned resin composition layer and the lid, and the sectional diameter thereof is selected appropriately in accordance with the purpose. The channel may include a reservoir in which the fluid is reserved. One end of the channel is connected to the inlet and the other to the outlet. The inlet and the outlet through which the sample is introduced and discharged are provided in the support or the lid. The channel may be branched, or the channels may merge together in the device. A plurality of inlets or outlets may be provided. The number of the inlets and that of the outlets may be different from each other.

Other features such as a reservoir tank, a pumping mechanism, a valve mechanism and a membrane separation mechanism may be provided. Further, the microfluidic device may be equipped with a temperature control mechanism that controls the temperature in the channel.

The support and the lid may be provided with electrodes and conductor units for establishing connections to detection elements. The electrodes and the conductor units are designed to be electrically connected to reading units that are engaged with housings in which the microfluidic devices of the invention are accommodated. These features are made of conductive materials and are in an embodiment metal films formed by a known production step such as photolithography, screen printing or sputtering. Examples of the conductive materials include gold, indium tin oxide (ITO), silver, platinum, palladium, aluminum, copper and titanium nitride. These materials may be used singly or as a mixture.

The microfluidic devices having the mechanisms described above may be used as, for example, microreaction elements (microreactors) for fields such as chemistry and biochemistry, DNA analysis elements, microelectrophoretic elements, microchromatographic elements, microelements for the preparation of samples for analyses such as mass spectroscopy and liquid chromatography, elements for physiochemical treatments such as extraction, membrane separation and dialysis, and microarray spotters.

### EXAMPLES

Hereinbelow, the present invention will be described in greater detail based on Examples without limiting the scope of the invention to such Examples.

### 1. Preparation of photosensitive resin compositions

### [Example 1]

A flask was charged with 70 g of propylene glycol monomethyl ether acetate, which was heated to 80°C. To the flask, a solution including 4 g of methacrylic acid, 20 g of p-hydroxystyrene, 70 g of 3-ethyl-3-acryloxymethyloxetane, 16 g of styrene, 32 g of n-butyl acrylate, 70 g of propylene glycol monomethyl ether acetate and 3 g of azoisobutyronitrile was added dropwise over a period of 2 hours. The mixture was heated at 80°C for 5 hours and was heated at 100°C for 1 hour. The resultant solution was reprecipitated to give an oxetanyl group-containing polymer (a polymer A) (polystyrene-equivalent Mw measured by GPC: 18,000). ¹³C-NMR confirmed that the polymer included the structural units derived from the respective monomers in a mass ratio consistent with the amounts in which the monomers had been added.

100 Parts by mass of the polymer A as an oxetanyl compound, 2 parts by mass of 4-(phenylthio)phenyldiphenylsulfonium tris(pentafluoroethyl)trifluorophosphate as a photoacid generator, 10 parts by mass of trimethylolpropane triglycidyl ether and 3 parts by mass of glycidoxypropyl trimethoxysilane as oxiranyl compounds, and 0.1 part by mass of "FTX-218" manufactured by NEOS COMPANY LIMITED as a fluorine surfactant were dissolved in 250 parts by mass of propylene glycol monomethyl ether acetate to give a uniform solution. A photosensitive resin composition of Example 1 was thus obtained.

### [Example 2]

100 Parts by mass of the polymer A as an oxetanyl compound, 2 parts by mass of 4-(phenylthio)phenyldiphenylsulfonium tris(pentafluoroethyl)trifluorophosphate as a photoacid generator, 10 parts by mass of trimethylolpropane triglycidyl ether and 3 parts by mass of glycidoxypropyl trimethoxysilane as oxiranyl compounds, 0.1 part by mass of "FTX-218" manufactured by NEOS COMPANY LIMITED as a fluorine surfactant, and 0.05 parts by mass of N-t-butoxycarbonyl-2-phenylbenzimidazole as an acid diffusion-controlling agent were dissolved in 250 parts by mass of propylene glycol monomethyl ether acetate to give a uniform solution. A photosensitive resin composition of Example 2 was thus obtained.

### [Comparative Example 1]

A flask was charged with 70 g of propylene glycol monomethyl ether acetate, which was heated to 80°C. To the flask, a solution including 4 g of methacrylic acid, 20 g of p-hydroxystyrene, 60 g of glycidoxypropyl methacrylate, 16 g of styrene, 32 g of n-butyl acrylate, 70 g of propylene glycol monomethyl ether acetate and 3 g of azoisobutyronitrile was added dropwise over a period of 2 hours. The mixture was heated at 80°C for 5 hours and was heated at 100°C for 1 hour. The resultant solution was reprecipitated to give an epoxy group-containing polymer B (polystyrene-equivalent Mw measured by GPC: 16,000). ¹³C-NMR confirmed that the polymer included the structural units derived from the respective monomers in a mass ratio consistent with the amounts in which the monomers had been added.

A photosensitive resin composition of Comparative Example 1 was obtained using the same amounts of the components as described in Example 1 except that the polymer B was used in place of the polymer A.

### 2. Production of microfluidic devices

### [Example 3]

Fig. 2 schematically illustrates a microfluidic device produced in Example 3.

The photosensitive resin composition of Example 1 was spin coated onto an 8-inch silicon wafer having semiconductor sensors. The coating was heated with a hot plate at 110 °C for 3 minutes to form a film having a thickness of 30 µm. The film was irradiated (apparatus: "NSR2005 i10D" manufactured by Nikon Corporation, 1000 µJ/cm²) through a mask having light-shielding sections, and was thereafter heated at 110°C for 5 minutes. The latent images were then developed with an aqueous solution containing 2.38 mass% of tetramethylammonium hydroxide. Fig. 2 (b) shows the pattern as seen from above the film. Thereafter, the silicon wafer having the patterned film was diced into 2 cm x 2 cm chips each having the patterned film. While heating the chip at 200°C, a glass plate 300 (2 cm in length, 2 cm in width, 1 mm in thickness) was placed and bonded to the chip with use of a die bonder (apparatus: "FTD-1940" manufactured by SHIBAURA MECHATRONICS CORPORATION). As illustrated in Fig. 2(a), the glass plate had apertures (an inlet 301 and an outlet 302) to allow a fluid containing a substrate to be introduced into and discharged out of the channel. Thereafter, the stack was heated at 180°C for 2 hours. A microfluidic device was thus produced.

The microfluidic device produced was cut and the cross section of the channel was observed with an electron microscope. The channel was found to have satisfactory walls (Fig. 2(c)). Fig. 2 (c) illustrates the cross section viewed along line A-B in Fig. 2(b).

After an aqueous solution containing 1 mass% of ethidium bromide was passed through the channel of the microfluidic device, the walls of the channel were inspected and were found to be stain-free. This result showed that the channel was highly resistant to the attachment of substrates.

### [Example 4]

A microfluidic device was produced in the same manner as in Example 3, except that the photosensitive resin composition of Example 1 was replaced by the photosensitive resin composition of Example 2. The channel was found to be highly resistant to the attachment of substrates.

### [Comparative Example 2]

A microfluidic device was produced in the same manner as in Example 3, except that the photosensitive resin composition of Example 1 was replaced by the photosensitive resin composition of Comparative Example 1. The microfluidic device produced was unsatisfactory in that the lid was not securely bonded.

Similarly to Example 3, the microfluidic device was cut and the cross section of the channel was observed with an electron microscope. The channel was found to have less smooth walls than the walls of the channel obtained in Example 2. Similarly to Example 2, an aqueous solution containing 1 mass% of ethidium bromide was passed through the channel of the microfluidic device. The walls of the channel were stained.

### REFERENCE SIGNS LIST

10: SUPPORT, 20: RESIN COMPOSITION LAYER, 21: PATTERNED RESIN COMPOSITION LAYER, 30: LID, 40: GROOVE, 41: CHANNEL, 50: MASK PATTERN, 51: EXPOSURE LIGHT, 100: SUPPORT HAVING SEMICONDUCTOR SENSOR, 200: PATTERNED PHOTOSENSITIVE RESIN COMPOSITION LAYER, 201: CHANNEL, 300: GLASS PLATE, 301: INLET, 302: OUTLET

## Claims

1. A microfluidic device including a channel in a body,
the channel having a patterned resin composition layer formed from a photosensitive resin composition comprising an oxiranyl group-containing compound, an oxetanyl group-containing compound and a photoacid generator.

2. A microfluidic device including a channel in a body,
the channel being defined by a support; a patterned resin composition layer formed from a photosensitive resin composition comprising an oxiranyl group-containing compound, an oxetanyl group-containing compound and a photoacid generator; and a lid covering a groove defined by the support and the patterned resin composition layer.

3. The microfluidic device according to claim 1 or 2, wherein the photosensitive resin composition further comprises an acid diffusion-controlling agent.

4. The microfluidic device according to claim 2, wherein at least one selected from the support and the lid has a detection element.

5. A process for producing a microfluidic device including a channel in a body, the process comprising:
a step of forming a resin composition layer on a support, the resin composition layer including a photosensitive resin composition including an oxiranyl group-containing compound, an oxetanyl group-containing compound and a photoacid generator,
a step of selectively irradiating and developing the resin composition layer, and
a step of arranging a lid on the developed resin composition layer.

6. A channel-forming photosensitive resin composition used for the formation of a channel in a body of a microfluidic device,
the channel-forming photosensitive resin composition comprising an oxiranyl group-containing compound, an oxetanyl group-containing compound and a photoacid generator.
